# EUROPÄISCHE PATENTSCHRIFT

(11) **EP 2 007 347 B1**
(45) Veröffentlichungstag und Bekanntmachung des Hinweises auf die Patenterteilung: **28.11.2018**
(21) Anmeldenummer: 07711695.2
(22) Anmeldetag: 27.02.2007
(51) Int. Cl.: A61K 9/00, C02F 1/68, A61K 9/46, A61K 9/16, C02F 103/02

(54) **ZUSAMMENSETZUNG UMFASSEND EIN MITTEL ZUM STABILISIEREN VON WIRKSTOFFEN IM TRINKWASSER UND EINE BRAUSEMISCHUNG, VERFAHREN ZU DEREN HERSTELLUNG UND DEREN VERWENDUNG**
COMPOSITION COMPRISING AN AGENT FOR STABILIZING ACTIVE SUBSTANCES IN DRINKING WATER, EFFERVESCENT MIXTURE, METHOD FOR THE PRODUCTION THEREOF, AND USE THEREOF
COMPOSITION COMPRENANT UN MOYEN POUR STABILISER DES PRINCIPES ACTIFS DANS L'EAU POTABLE, MÉLANGE EFFERVESCENT, MÉTHODE DE PRÉPARATION ET UTILISATION DE LA MÊME

(30) Priorität: 28.02.2006 DE 102006009338; 19.05.2006 DE 102006023578; 17.11.2006 DE 102006054260
(43) Veröffentlichungstag der Anmeldung: 31.12.2008
(73) Patentinhaber: Lohmann Animal Health GmbH, 27472 Cuxhaven (DE)
(72) Erfinder: SCHWARZ, Klaus-Uwe, 27476 Cuxhaven (DE); NESTLER, Helge, 84508 Burgkirchen (DE); BRÖCKEL, Ulrich, 67251 Freinsheim (DE); IBURG, Michael, 28359 Bremen (DE)
(74) Vertreter: Bassinder, Emma Marie
(86) Internationale Anmeldenummer: PCT/EP2007/001679
(87) Internationale Veröffentlichungsnummer: WO 2007/098924

(56) Entgegenhaltungen:
- EP-A2- 0 377 906
- WO-A-00/07571
- WO-A-91/03241
- WO-A-97/44017
- WO-A2-02/067846
- DE-A1- 3 933 164
- DE-A1- 19 700 368
- FR-A1- 2 793 685
- GB-A- 1 165 098
- GB-A- 1 221 038

## Beschreibung

Die Erfindung bezieht sich auf eine Wasser stabilisierende Zusammensetzung, auf ein Verfahren zu Herstellung einer Wasser stabilisierenden Zusammensetzung und auf die Verwendung einer Wasser stabilisierenden Zusammensetzung.

In der Veterinärmedizin, vor allem im Bereich der Schweine- und Geflügelproduktion, werden vermehrt Lebendimpfstoffe über das Trinkwasser appliziert. Diese Art der Verabreichung führt zur sicheren und guten Immunisierung der Tiere, da sie den natürlichen Weg vieler Infektionen nachahmt und somit sämtliche Teile der Immunantwort stimuliert. Gleichzeitig ist die Gabe von Lebendimpfstoffen über das Trinkwasser eine ökonomisch sinnvolle und aus Tierschutzgründen zu bevorzugende Art der Impfung.

Dem Wasser kommt hierbei eine besondere Bedeutung zu, da es gleichzeitig das Lösungs- und das Transportmittel für die Lebendimpfstämme ist. Die Wasserqualität entscheidet letztlich über das Überleben der Impfstoffe auf ihrem Weg bis zum Tier. Die Wasserqualität kann jedoch sehr unterschiedlich sein und insbesondere in Abhängigkeit vom Produktionsbetrieb, der Wasserversorgung und der Zeit schwanken. Dies kann die Wirksamkeit von Lebendimpfstoffen verändern bzw. beeinträchtigen.

Zur Stabilisierung von Lebendimpfstoffen wird dem Trinkwasser häufig Magermilchpulver zugesetzt. Magermilchpulver löst sich aber sehr schlecht im Trinkwasser, so daß es zu Verstopfungen des Tränkesystems kommen kann.

Ferner sind Tabletten bekannt, die außer einem Mittel zur Wasserstabilisierung einen blauen Farbstoff enthalten, der dem Anwender die Verbreitung des Wasserstabilisators und des Impfstoffes über das Tränkesystem und die Aufnahme durch das Tier indiziert. Die Anwendung ist jedoch wegen der ungünstigen Auflösungseigenschaften der Tabletten mühselig.

Ferner bekannt ist ein Pulver, das ein Mittel zum Stabilisieren von Wasser und einen blauen Farbstoff enthält. Das Pulver staubt sehr und ist äußerst schlecht in Wasser einmischbar. Die Bestandteile neigen dazu aufzuschwimmen oder zu sedimentieren, so daß stark umgerührt werden muß. Nicht aufgelöste Partikel können das Tränkesystem verstopfen.

Die WO 02/067846 A2 beschreibt Brausezubereitungen von Impfstoffen zum tierärztlichen Gebrauch. Es besteht die Gefahr, dass die Impfstoffe im Trinkwasser destabilisiert werden.

Davon ausgehend liegt der Erfindung die Aufgabe zugrunde, eine anwendungsfreundlichere Wasser stabilisierende Zusammensetzung, ein Verfahren zur Herstellung der Wasser stabilisierenden Zusammensetzung und eine Verwendung der Wasser stabilisierenden Zusammensetzung zur Verfügung zu stellen.

Die Aufgabe wird durch eine Wasser stabilisierende Zusammensetzung mit den Merkmalen des Anspruches 1 gelöst. Vorteilhafte Ausgestaltungen der Zusammensetzung sind in den Unteransprüchen 2 bis 11 angegeben. Eine Kombination einer Flasche mit der Zusammensetzung ist in den Unteransprüchen 12 und 13 angegeben.

Die erfindungsgemäße Wasser stabilisierende Zusammensetzung umfaßt ein Mittel zum Stabilisieren von Wirkstoffen im Trinkwasser und eine Brausemischung wobei das Stabilisierungsmittel einen Komplexbildner enthält, wobei der Komplexbildner Magermilch ist und wobei die Zusammensetzung ein Feststoff in Form eines Granulats ist.

Bei der Brausemischung handelt es sich um eine Mischung, die beim Auflösen in Wasser ein Gas entwickelt und stark aufbraust. Aufgrund der enthaltenen Brausemischung braust die Wasser stabilisierende Zusammensetzung beim Eingeben in das Trinkwasser auf, wodurch die Auflösung und Verteilung des Mittels zum Stabilisieren von Wirkstoffen im Trinkwasser intensiviert wird. Infolgedessen kommt es zu einer besonders schnellen und gleichmäßigen Einmischung des Mittels zum Stabilisieren von Wirkstoffen in das Trinkwasser. Ein Einrühren der Zusammensetzung in das Trinkwasser ist nicht erforderlich bzw. der Aufwand für das Einrühren kann beträchtlich reduziert werden. Verunreinigungen des Tränkesystems, die dessen Funktion beeinträchtigen, werden vermieden. Die Zusammensetzung ist infolgedessen besonders anwendungsfreundlich.

Gemäß einer Ausgestaltung umfaßt das Mittel zum Stabilisieren weiterhin mindestens einen der folgenden Bestandteile: einen weiteren Komplexbildner, ein Reduktionsmittel, ein Puffermittel, einen Farbstoff.

Ein Komplexbildner ist in der Lage, im Trinkwasser enthaltene Schwermetalle zu inaktivieren, die anderenfalls Lebendimpfstoffe oder andere Wirkstoffe destabilisieren. Ein Reduktionsmittel neutralisiert ein Oxidationsmittel, das im Trinkwasser beispielsweise in Form von Rückständen von Reinigungsmitteln enthalten sein kann, die ebenfalls Lebendimpfstoffe und andere Wirkstoffe destabilisieren können. Ein Puffermittel puffert den pH-Wert auf einen für Lebendimpfstoffe oder andere Wirkstoffe besonders günstigen Wert. Ein Farbstoff ermöglicht die optische Kontrolle des Tränkesystems auf Befüllung mit wirkstoffhaltigem Wasser. Ferner soll der Farbstoff durch nicht-permanente Färbung von Schnabel, Rachen und Zunge der Tiere die Aufnahme wirkstoffhaltigen Wassers kontrollierbar machen. Gleichzeitig wertet der Farbstoff die Wasser stabilisierende Zusammensetzung und das eingefärbte Trinkwasser optisch auf. Die Farbe ist z.B. blau oder grün.

Gemäß einer Ausgestaltung ist der weitere Komplexbildner ausgewählt aus mindestens einer der folgenden Substanzen: Zitronensäure, Thiosulfat, Adipinsäure und Benzoesäure.

Gemäß einer Ausgestaltung ist das Reduktionsmittel ausgewählt aus mindestens einer der folgenden Substanzen: Thiosulfat und Lactose.

Gemäß einer Ausgestaltung ist das Puffermittel ausgewählt aus mindestens einer der folgenden Substanzen: Zitronensäure, Hydrogencarbonat (Bicarbonat), Natriumbicarbonat, Natriumcarbonat, Carbonat.

Gemäß einer Ausgestaltung ist der Farbstoff ein Lebensmittelfarbstoff. Die Aufnahme von Lebensmittelfarbstoff durch das Tier ist unbedenklich.

Gemäß einer Ausgestaltung umfaßt die Brausemischung Hydrogencarbonat und eine organische Säure. Beim Auflösen in Wasser entwickelt die organische Säure aus Hydrogencarbonat oder Carbonat Kohlendioxid. Das Hydrogencarbonat ist z.B. Natriumhydrogencarbonat. Die organische Säure ist beispielsweise Zitronensäure, Benzoesäure, Adipinsäure oder Weinsäure. Hydrogencarbonat kann gleichzeitig als Reduktionsmittel und Puffermittel und die organische Säure kann gleichzeitig als Komplexbildner und Puffermittel dienen.

Die Wasser stabilisierende Zusammensetzung kann grundsätzlich in flüssiger oder in fester Form vorliegen. Gemäß einer Ausgestaltung ist die Wasser stabilisierende Zusammensetzung fest. Sie kann z.B. tablettenförmig oder pulverförmig oder granulatförmig sein. In beiden Fällen wird durch die Brausemischung die Auflösung und das Einmischen der Wasser stabilisierenden Zusammensetzung verbessert. Gemäß einer Ausgestaltung hat die Wasser stabilisierende Zusammensetzung die Form eines Granulates. Granulate haben gegenüber Tabletten oder einem Pulver Handhabungsvorteile. Sie lassen sich leichter fördern und dosieren. Ferner kann ein Granulat im wesentlichen staubfrei bereitgestellt werden.

Gemäß einer weiteren Ausgestaltung umfaßt das Granulat Partikel bzw. Granulate, welche sowohl das Mittel zum Stabilisieren als auch die Brausemischung enthalten. Beim Auflösen im Wasser sprengt das von der Brausemischung freigesetzte Gas die Granulatstruktur und an den Granulatkörnchen anhaftende Blasen ziehen ein Aufschwimmen der Partikel bzw. Granulate nach sich. Dieser Effekt gewährleistet eine schnelle Durchmischung des Mittels zum Stabilisieren von Wirkstoffen mit dem Trinkwasser.

Gemäß einer Ausgestaltung umfaßt das Granulat Partikel aus unter Druck verfestigtem Pulver. Die Partikel sind unter Druck aus einem pulverförmigen Mittel zum Stabilisieren oder seinen pulverförmigen Bestandteilen und einer pulverförmigen Brausemischung oder ihren pulverförmigen Bestandteilen zusammensetzbar, so daß sie das Mittel zum Stabilisieren und die Brausemischung umfassen.

Gemäß einer Ausgestaltung umfaßt die Zusammensetzung Partikel bzw. Granulate aus unter Druck verfestigtem Pulver mit einer Partikelgröße des Pulvers von maximal etwa 100 µm. Gemäß einer weiteren Ausgestaltung haben die Partikel der Zusammensetzung eine Größe von mindestens etwa 300 µm und maximal etwa 3 mm. Der genannte Partikelgrößenbereich ist für die Anwendung des Granulates besonders vorteilhaft.

Gemäß einer Ausgestaltung weist eine Kombination aus einer Flasche und einer darin angeordneten Wasser stabilisierenden Zusammensetzung der vorstehend erläuterten Art an der Flasche eine Verschlußkappe mit einem definierten Nennvolumen auf. Die Verschlußkappe kann zum Eindosieren der Wasser stabilisierenden Zusammensetzung in ein Tränkesystem genutzt werden. Außerdem kann sie dem dichten Abschluß der Flasche dienen.

Hierzu ist die Verschlußkappe gemäß einer Ausgestaltung eine mit der Flasche verschraubte Schraubkappe.

Ferner wird die Aufgabe durch ein Verfahren zur Herstellung mit den Merkmalen des Anspruches 14 gelöst. Vorteilhafte Ausgestaltungen des Verfahrens sind in den Unteransprüchen 15 bis 20 angegeben.

Bei dem erfindungsgemäßen Verfahren zur Herstellung einer Wasser stabilisierenden Zusammensetzung der vorerwähnten Art wird ein Mittel zum Stabilisieren von Wirkstoffen und eine Brausemischung in Pulverform gemischt und wird die Mischung kompaktiert bzw. granuliert.

Nach dem Verfahren werden Pulver zu Granulat mit den oben erwähnten Anwendungsvorteilen verarbeitet. Es können sämtliche Bestandteile des Mittels zum Stabilisieren und der Brausemischung in Pulverform zugegeben, gemischt und kompaktiert werden. Es ist aber auch möglich, ein vorgefertigtes Mittel zum Stabilisieren von Wirkstoffen in Pulverform mit einer vorgefertigten Brausemischung in Pulverform zu mischen und die Mischung zu kompaktieren bzw. zu granulieren.

Für das Mischen der Pulver kann eine Vielzahl gängiger Rührwerke oder anderer Mischertypen eingesetzt werden, die beim Mischen von Pulvern zum Einsatz kommen. Ebenso können für das Kompaktieren bzw. Granulieren beliebige Vorrichtungen zum Kompaktieren bzw. Granulieren von Pulvern Verwendung finden (wie z.B. Tablettenpresse, Kollergang, Intensivmischer).

Gemäß einer Ausgestaltung wird mittels eines Doppelwalzenkompaktierers kompaktiert. Zum Kompaktieren kann auch ein Extruder eingesetzt werden.

Gemäß einer weiteren Ausgestaltung wird das Kompaktat gebrochen (z.B. mittels Kollergang), um eine gewünschte Partikelgrößenverteilung herzustellen. Gemäß einer weiteren Ausgestaltung wird das gebrochene Kompaktat gesiebt und der Siebdurchgang der Mischung dem Pulver zugeführt. Durch Aussiebung und Rückführung der Feinanteile des gebrochenen Kompaktates in den Kompaktierungsprozeß werden feinkörnige Bestandteile des Granulats vermieden. Das resultierende Granulat ist im wesentlichen staubfrei.

Schließlich wird die Aufgabe durch eine Verwendung gemäß Anspruch 21 gelöst. Vorteilhafte Ausgestaltungen der Verwendung sind in den Unteransprüchen 22 und 23 angegeben.

Die erfindungsgemäße Verwendung einer Wasser stabilisierenden Zusammensetzung der vorerwähnten Art erfolgt zum Stabilisieren der Gabe von Wirkstoffen über Trinkwasser.

Gemäß einer Ausgestaltung werden die Wasser stabilisierende Zusammensetzung und die Wirkstoffe an derselben Aufgabestelle in ein Tränkesystem eingemischt. Die Aufgabestelle ist beispielsweise ein Vorlaufbehälter oder ein Reservoir, in dem die Mischung der Wasser stabilisierenden Zusammensetzung und der Wirkstoffe mit dem Trinkwasser erfolgt. Die Aufgabestellung kann aber auch ein Rohrstück in einem Leitungssystem sein.

Die Wasser stabilisierende Zusammensetzung kann mit verschiedenen Wirkstoffen zur Anwendung kommen. Gemäß einer Ausgestaltung sind die Wirkstoffe Lebendimpfstoffe oder Totimpfstoffe oder Probiotika. Die Lebendimpfstoffe sind z.B. Salmonella-, Influenza-, Anämia- oder Geflügelpest-Impfstoffe.

Nachfolgend wird die Erfindung anhand von Ausführungsbeispielen näher erläutert, die sich auf die anliegenden Zeichnungen beziehen. In den Zeichnungen zeigen:
- Fig. 1: eine Anlage zum Herstellen der Wasser stabilisierenden Zusammensetzung in einer grobschematischen Skizze;
- Fig. 2: eine Flasche mit einer Verschlußkappe zum Aufbewahren und Dosieren der Wasser stabilisierenden Zusammensetzung in Seitenansicht;
- Fig. 3: ein Tränkesystem für die Aufgabe einer Wasser stabilisierenden Zusammensetzung gemeinsam mit einem Wirkstoff.

In der nachfolgenden Tabelle sind für ein Ausführungsbeispiel (2 Alternativen) der Wasser stabilisierenden Zusammensetzung die Komponenten und deren Gewichtsanteile:

| Komponente | Anteil |
|---|---|
| Na-Thiosulfat | 17,8 % |
| Magermilch-Pulver | 10,0 % |
| Zitronensäure | 9,0 % |

| Natriumhydrogencarbonat | |
|---|---|
| Alternative 1: | 18% |
| Alternative 2: | 20% |
| Lactosemonohydrat | 35,7 % |
| Patentblau 5 (E131) | 2,0 % |

| Natriumcarbonat | |
|---|---|
| Alternative 1: | 2% |
| Alternative 2: | 0% |
| Magnesiumstearat | 0,5 % |
| PEG 6000 Polyethylenglycol molares Gewicht 6000 g/mol | 5,0 % |

Die obigen Komponenten werden in Pulverform eingesetzt.

Alternative 1 enthält Natriumhydrogencarbonat und Natriumcarbonat. Alternative 2 enthält Natriumhydrogencarbonat und kein Natriumcarbonat.

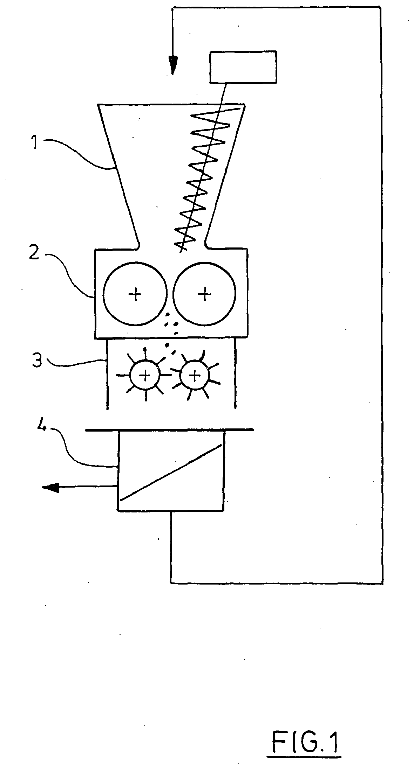

Die Verarbeitung kann in einer Anlage gemäß Fig. 1 erfolgen. Die Pulver werden von oben einem nach unten konisch verjüngten Rührwerksmischer 1 zugeführt. Durch den Ausgang an der Unterseite des Rührwerksmischers 1 gelangen die vorgemischten Pulver in einen Doppelwalzenkompaktierer 2. Dieser ist grundsätzlich wie eine Walzen-Brikettierpresse aufgebaut.

Die Bestandteile der Mischung sind im wesentlichen gleichmäßig über sämtliche Partikel des Kompaktats verteilt.

Unten wird das Kompaktat aus dem Doppelwalzen-Kompaktierer 2 in einen Brecher 3 ausgetragen. Dort wird das Kompaktat zu kleineren Partikeln gebrochen.

Aus dem Brecher 3 gelangt das Granulat in eine Siebvorrichtung 4. Der feine Siebdurchgang wird als Recyclat von der Sieb Vorrichtung 4 in den Mischer 1 zurückgeführt. Der Siebrückstand bildet das fertige Produkt.

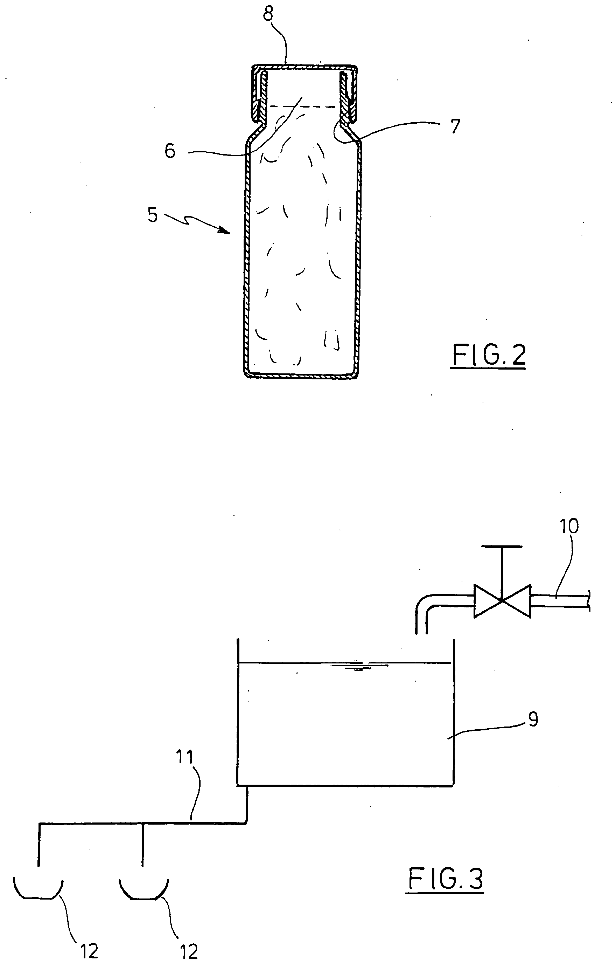

Gemäß Fig. 2 wird das Produkt in eine Flasche 5 gefüllt, die oben am Flaschenkörper um eine Öffnung 6 ein Schraubgewinde 7 aufweist, auf das eine Schraubkappe 8 aufgeschraubt wird. Die Schraubkappe 8 ist im wesentlichen topfförmig. Ihr Hohlraum weist ein bestimmtes Nennvolumen auf und kann zum Dosieren des Granulats herangezogen werden.

Gemäß Fig. 3 umfaßt ein Tränkesystem einen Vorlagebehälter 9 für Trinkwasser, dem eine Einspeisung 10 für Trinkwasser zugeordnet ist. Der Vorlagebehälter 9 ist unten mit einem Leitungssystem 11 verbunden, das zu verschiedenen Abgabestellen 12 in Form von Wassertrögen verzweigt ist. An den Abgabestellen 12 können beispielsweise Hühner oder anderes Geflügel Trinkwasser aufnehmen.

In den Vorlagebehälter 9 werden dem Trinkwasser die Wasser stabilisierende Zusammensetzung und ein Impfstoff zugemischt. Durch die Wirkung der Brausemischung wird im Vorlaufbehälter 9 eine gleichmäßige Verteilung der Zusammensetzung und des Impfstoffes erreicht, so daß die Abgabestellen 12 gleichmäßig mit Zusammensetzung und Impfstoff versorgt werden. Somit ist die Impfstoffaufnahme anhand der Blaufärbung der Schnäbel der Tiere erkennbar.

## Patentansprüche

1. Wasser stabilisierende Zusammensetzung umfassend ein Mittel zum Stabilisieren von Wirkstoffen im Trinkwasser und eine Brausemischung, wobei das Stabilisierungsmittel einen Komplexbildner enthält, wobei der Komplexbildner Magermilch ist und wobei die Zusammensetzung ein Feststoff in Form eines Granulats ist.

2. Zusammensetzung nach Anspruch 1, wobei das Stabilisierungsmittel weiterhin mindestens einen der folgenden Bestandteile umfasst:
• einen weiteren Komplexbildner
• ein Reduktionsmittel
• ein Puffermittel
• einen Farbstoff.

3. Zusammensetzung nach Anspruch 2, wobei der weitere Komplexbildner aus mindestens einer der folgenden Substanzen ausgewählt ist: Zitronensäure, Thiosulfat, Adipinsäure und Benzoesäure.

4. Zusammensetzung nach Anspruch 2 oder 3, wobei das Reduktionsmittel aus mindestens einer der folgenden Substanzen ausgewählt ist: Thiosulfat, Lactose.

5. Zusammensetzung nach einem der Ansprüche 2 bis 4, wobei das Puffermittel aus mindestens einer der folgenden Substanzen ausgewählt ist: Zitronensäure, Hydrogencarbonat (Bicarbonat), Natriumbicarbonat, Natriumcarbonat, Carbonat.

6. Zusammensetzung nach einem der Ansprüche 2 bis 5, wobei der Farbstoff ein Lebensmittelfarbstoff ist.

7. Zusammensetzung nach einem der Ansprüche 1 bis 6, wobei die Brausemischung Hydrogencarbonat oder Carbonat und eine organische Säure umfasst.

8. Zusammensetzung nach einem der Ansprüche 1 bis 7, wobei das Granulat Partikeln umfasst, die sowohl das Stabilisierungsmittel als auch die Brausemischung enthalten.

9. Zusammensetzung nach einem der Ansprüche 1 bis 8, wobei das Granulat Partikeln aus unter Druck verfestigtem Pulver umfasst.

10. Zusammensetzung nach Anspruch 9 umfassend Partikeln aus unter Druck verfestigtem Pulver, wobei das Pulver eine Partikelgröße von maximal etwa 100 µm aufweist.

11. Zusammensetzung nach Anspruch 9 oder 10, wobei die Partikeln der Zusammensetzung eine Größe zwischen minimal etwa 300 µm und maximal etwa 3 mm aufweisen.

12. Kombination aus einer Flasche und, darin enthalten, einer Wasser stabilisierenden Zusammensetzung nach einem der Ansprüche 1 bis 11, wobei die Flasche eine Verschlusskappe mit einem definierten Nennvolumen aufweist.

13. Kombination nach Anspruch 12, wobei die Verschlusskappe eine auf die Flasche aufgeschraubte Schraubkappe ist.

14. Verfahren zur Herstellung einer Wasser stabilisierenden Zusammensetzung nach einem der Ansprüche 1 bis 11, wobei
• Magermilch und eine Brausemischung in Pulverform gemischt werden und
• die Mischung kompaktiert wird.

15. Verfahren nach Anspruch 14, wobei das Kompaktieren mit einem Doppelwalzenkompaktierer durchgeführt wird.

16. Verfahren nach Anspruch 14 oder 15, wobei das Kompaktieren mit einem Extruder durchgeführt wird.

17. Verfahren nach einem der Ansprüche 14 bis 16, wobei das Kompaktieren mit einer Tablettenpresse durchgeführt wird.

18. Verfahren nach einem der Ansprüche 14 bis 17, wobei das Kompaktat gebrochen wird.

19. Verfahren nach Anspruch 18, wobei das Brechen mit einem Kollergang durchgeführt wird.

20. Verfahren nach einem der Ansprüche 14 bis 19, wobei das gebrochene Kompaktat gesiebt wird und das durch das Sieb durchgehende Material zur Pulvermischung zurückgeführt wird.

21. Verwendung einer Wasser stabilisierenden Zusammensetzung nach einem der Ansprüche 1 bis 11 zum Stabilisieren der Gabe von Wirkstoffen über Trinkwasser.

22. Verwendung nach Anspruch 21, wobei die Wasser stabilisierende Zusammensetzung und die Wirkstoffe an derselben Aufgabestelle in ein Tränkesystem eingemischt werden.

23. Verwendung nach Anspruch 21 oder 22, wobei die Wirkstoffe Tot- oder Lebendimpfstoffe oder ein Probiotikum sind.

## Claims

1. Water-stabilizing composition comprising an agent for stabilizing active substances in drinking water and an effervescent mixture, wherein the stabilizing agent comprises a complexing agent, wherein the complexing agent is skim milk and wherein the composition is a solid in the form of a pellet.

2. Composition according to claim 1, wherein the stabilizing agent further comprises at least one of the following components:
• a further complexing agent
• a reducing agent
• a buffering agent
• a colorant.

3. Composition according to claim 2, wherein the further complexing agent is selected from at least one of the following substances: citric acid, thiosulfate, adipic acid and benzoic acid.

4. Composition according to claim 2 or 3, wherein the reducing agent is selected from at least one of the following substances: thiosulfate, lactose.

5. Composition according to one of the claims 2 through 4, wherein the buffering agent is selected from at least one of the following substances: citric acid, hydrogen carbonate (bicarbonate), sodium bicarbonate, sodium carbonate, carbonate.

6. Composition according to one of the claims 2 through 5, wherein the colorant is a food colorant.

7. Composition according to one of the claims 1 through 6, wherein the effervescent mixture comprises hydrogen carbonate or carbonate and an organic acid.

8. Composition according to one of the claims 1 through 7, wherein the pellet comprises particles containing both the stabilizing agent and the effervescent mixture.

9. Composition according to one of the claims 1 through 8, wherein the pellet comprises particles of powder solidified under pressure.

10. Composition according to claim 9 comprising particles of powder solidified under pressure, the powder having a particle size of a maximum of approximately 100 µm.

11. Composition according to claim 9 or 10, wherein the particles of the composition range between a minimum of approximately 300 µm and a maximum of approximately 3 mm.

12. Combination of a bottle and, contained therein, a water-stabilizing composition according to one of the claims 1 through 11, wherein the bottle has a seal cap with a defined nominal volume.

13. Combination according to claim 12, wherein the seal cap is a screw cap screwed onto the bottle.

14. Method for producing a water-stabilizing composition according to one of the claims 1 through 11, wherein
• skim milk and an effervescent mixture are mixed in powder form and
• the mixture is compacted.

15. Method according to claim 14, wherein compacting is accomplished with a tandem roller compactor.

16. Method according to claim 14 or 15, wherein compacting is accomplished with an extruder.

17. Method according to one of the claims 14 through 16, wherein compacting is accomplished with a tablet press.

18. Method according to one of the claims 14 through 17, wherein the compacted product is broken.

19. Method according to claim 18, wherein breaking is accomplished with a pan mill.

20. Method according to one of the claims 14 through 19, wherein the broken compacted product is sifted and the material passing through the sieve is returned to the powder mixture.

21. Use of a water-stabilizing composition according to one of the claims 1 through 11 for stabilizing the administration of active substances through drinking water.

22. Use according to claim 21, wherein the water-stabilizing composition and the active substances are mixed into a drinking system at the same dispensing point.

23. Use according to claim 21 or 22, wherein the active substances are dead or living vaccines or a probiotic.

## Revendications

1. Composition pour stabiliser l'eau, comprenant un agent pour stabiliser des principes actifs dans de l'eau potable et un mélange effervescent, l'agent de stabilisation contenant un complexant, le complexant étant le lait écrémé et la composition étant un solide sous forme d'un granulat.

2. Composition selon la revendication 1, l'agent de stabilisation comprenant en outre au moins un des constituants suivants :
- un autre complexant
- un agent de réduction
- un tampon
- un colorant.

3. Composition selon la revendication 2, l'autre complexant étant choisi parmi au moins l'une des substances suivantes : acide citrique, thiosulfate, acide adipique et acide benzoïque.

4. Composition selon la revendication 2 ou 3, l'agent de réduction étant choisi parmi au moins l'une des substances suivantes : thiosulfate, lactose.

5. Composition selon l'une quelconque des revendications 2 à 4, le tampon étant choisi parmi au moins l'une des substances suivantes : acide citrique, hydrogénocarbonate (bicarbonate), bicarbonate de sodium, carbonate de sodium, carbonate.

6. Composition selon l'une quelconque des revendications 2 à 5, le colorant étant un colorant alimentaire.

7. Composition selon l'une quelconque des revendications 1 à 6, le mélange effervescent comprenant de l'hydrogénocarbonate ou un carbonate et un acide organique.

8. Composition selon l'une quelconque des revendications 1 à 7, le granulat comprenant des particules qui contiennent tant l'agent de stabilisation que le mélange effervescent.

9. Composition selon l'une quelconque des revendications 1 à 8, le granulat comprenant des particules de poudre compactée sous pression.

10. Composition selon la revendication 9, comprenant des particules de poudre compactée sous pression, la poudre présentant une grosseur de particule d'au maximum environ 100 µm.

11. Composition selon la revendication 9 ou 10, les particules de la composition présentant une grosseur entre minimum environ 300 µm et maximum environ 3 mm.

12. Combinaison d'un flacon et d'une composition stabilisant l'eau qui y est contenue selon l'une quelconque des revendications 1 à 11, le flacon présentant un capuchon de fermeture d'un volume nominal défini.

13. Combinaison selon la revendication 12, le capuchon de fermeture étant un capuchon à visser, vissé sur le flacon.

14. Procédé pour la préparation d'une composition stabilisant l'eau selon l'une quelconque des revendications 1 à 11,
- du lait maigre et un mélange effervescent étant mélangés sous forme de poudre et
- le mélange étant compacté.

15. Procédé selon la revendication 14, le compactage étant réalisé à l'aide d'un compacteur à deux cylindres.

16. Procédé selon la revendication 14 ou 15, le compactage étant réalisé à l'aide d'une extrudeuse.

17. Procédé selon l'une quelconque des revendications 14 à 16, le compactage étant réalisé à l'aide d'une presse à comprimés.

18. Procédé selon l'une quelconque des revendications 14 à 17, le produit compacté étant concassé.

19. Procédé selon la revendication 18, le concassage étant réalisé à l'aide d'un broyeur à meules.

20. Procédé selon l'une quelconque des revendications 14 à 19, le produit compacté concassé étant tamisé et le matériau passant à travers le tamis étant recyclé dans le mélange poudreux.

21. Utilisation d'une composition stabilisant l'eau selon l'une quelconque des revendications 1 à 11 pour la stabilisation de l'administration de principes actifs via l'eau potable.

22. Utilisation selon la revendication 21, la composition stabilisant l'eau et les principes actifs étant mélangés au même point d'alimentation dans un système d'abreuvage.

23. Utilisation selon la revendication 21 ou 22, les principes actifs étant un vaccin mort ou vivant ou un probiotique.
